# EUROPEAN PATENT SPECIFICATION

(11) **EP 1 702 650 B1**
(45) Date of publication and mention of the grant of the patent: **21.05.2008**
(21) Application number: 06003976.5
(22) Date of filing: 27.02.2006
(51) Int. Cl.: A61N 5/10, A61B 6/04

(54) **Patient positioning system and method**
System und Verfahren zur Patientenpositionierung
Procédé et dispositif pour le positionnement d'un patient

(30) Priority: 14.03.2005 JP 2005071291
(43) Date of publication of application: 20.09.2006
(73) Proprietor: HITACHI, LTD., Chiyoda-ku Tokyo 100-8280 (JP)
(72) Inventor: Nagamine, Yoshihiko c/o Hitachi, Ltd. I.P. Office, Chiyoda-ku Tokyo 100-8220 (JP); Matsuda, Koji c/o Hitachi, Ltd. I.P. Office, Chiyoda-ku Tokyo 100-8220 (JP)
(74) Representative: Strehl Schübel-Hopf & Partner

(56) References cited:
- EP-A- 1 454 653
- US-A- 5 825 845
- US-A- 5 970 164
- US-A1- 2004 264 628
- US-B1- 6 307 914
- US-B1- 6 516 046

## Description

### BACKGROUND OF THE INVENTION

### 1. Field of the Invention

The present invention relates to a patient positioning system and a patient positioning method which are used in radiation treatment for irradiating various kinds of radiations, such as an X-ray and a particle beam represented by a proton beam, to an affected part of the body for treatment.

### 2. Description of the Related Art

Recently, radiation treatment has been more widely practiced aiming at bringing tumor cells to death by irradiating various kinds of radiations to the tumor cells. While an X-ray is most prevalently used as the radiation in such treatment, the treatment using a particle beam, such as a proton beam, is also practiced.

One of important processes in the radiation treatment is to make precise positioning of a patient. The term "patient positioning" generally means a process performed by a medical engineer or a doctor through the steps of comparing a DRR (Digital Reconstructed Radiograph) image which is outputted from a treatment planning unit with a DR (Digital Radiograph) image which is taken before the start of radiation irradiation by using an X-ray image capturing device while a patient is lying on a treatment bed, computing a deviation between the patient position decided in the treatment planning and the current patient position on the treatment bed, and determining a shift amount of the treatment bed and moving the treatment bed such that the two images are matched with each other.

The DRR image is an image simulating the DR image and is produced from a CT image taken in the treatment planning stage. Hereinafter, the DRR image and the DR image are referred to also as a reference image and a comparative image, respectively, as required. Note that, in the patient positioning process, an image taken by an X-ray simulator, etc. is sometimes used as the reference image instead of the DRR image.

Hitherto, the so-called reference point (computing point) input method has been used as a method for computing a deviation between the current patient position and the position decided in the treatment planning stage. That method is performed through the steps of displaying the reference image and the comparative image on a monitor of a computer, designating corresponding points (which are selected as clearly recognizable points, e.g., bone portions, the designated points being called reference points) in both the images with an system operator by using an input device, e.g., a mouse, for computing a shift amount of the treatment bed, and deciding the shift amount so that an error between those corresponding points is minimized (see, e.g., Patent Document 1: JP,A 2000-510023 (Figs. 6-7)).

Further, an image comparing method has also been proposed which comprises the steps of comparing pixel values of the reference image and the comparative image with each other, and deciding the shift amount so that, for example, the correlation coefficient or the square of the difference is minimized (see, e.g., Patent Document 2: JP,A 2004-267250 (Figs. 8-12)).

U.S Patent 5,825,845 discloses a patient positioning system and method with the features included in the first part of claim 1 and claim 14, respectivley.

### SUMMARY OF THE INVENTION

With recent development of the imaging technology, DR images also tend to have higher resolutions and larger sizes. For example, a DR image of 2048 × 2048 pixels is output from an X-ray image capturing device. When such a large-sized image is displayed in its entirety on an ordinary monitor (e.g., a monitor with resolution of 1280 × 1024 pixels), the entire image cannot be displayed in size of 100% (i.e., without scaling-down). In the above example of trying to display the image of 2048 × 2048 pixels on the monitor with resolution of 1280 × 1024 pixels, an image scaled down to 50% at maximum is displayed on the monitor.

When an operator inputs reference points in accordance with the above-mentioned reference point input method while looking at the whole of the DR image displayed on the ordinary monitor, the reference points are input on the scaled-down image as described above. As a result, the following drawback occurs. If the image is displayed in size of 100%, a particular point of 1 pixel can be designated. In the above example, however, because the image is scaled down to 50% in height and width, 4 pixels are selected once. Practically, because buttons, a scroll bar, a menu, etc. for a graphical user interface are also displayed on a monitor screen according to the image display software used, there is a possibility that an actual scaling factor of the displayed image is further reduced and the number of pixels selected once is further increased. Hence, the correspondence between the reference point intended by the operator and the corresponding point on the image becomes ambiguous, thus resulting in a fear that a different point on the image from the intended one is set as the reference point and accuracy in positioning the patient is deteriorated.

On the other hand, with the known image comparing method described above, since the computation is executed regardless of the display scaling factor, it is possible to avoid the above-mentioned problem of a reduction in the positioning accuracy, which is caused by ambiguousness in setting of the reference point. However, because of the process of comparing pixel values over entire areas of the images to be compared, the known image comparing method requires a larger amount of computations and takes a longer time than the known reference point input method.

Thus, any of the known reference point input method and the known image comparing method cannot achieve an improvement in the positioning accuracy and a cut in the positioning time at the same time.

With the view of solving the above-described problems in the related art, an object of the present invention is to provide a patient positioning system and a patient positioning method which can realize an improvement in the positioning accuracy and a cut in the positioning time at the same time.

The above object is achieved by the patient positioning system defined in claim 1 and corresponding method fefined in claim 14.

In an embodiment of the patient positioning system, the particular region containing the affected area in the patient's body is enlarged depending on the scaling-down factor used to display the initial image information, the representative region is set in the enlarged region, and the positioning information is produced such that the positionial deviation between the corresponding the representative region is minimized. It is therefore possible to avoid the problem of a reduction in positioning accuracy even when an operator designates the particular region (reference point) in a scaled-down display state. Further, because pixel values are not compared over the entire image unlike in the known image comparing method, the amount of computations is greatly reduced and the positioning time can be shortened. As a result, an improvement in positioning accuracy and a cut-down in positioning time can be realized at the same time.

### BRIEF DESCRIPTION OF THE DRAWINGS

Fig. 1 is a schematic view showing the overall configuration of a patient positioning system according to a first embodiment of the present invention;
Fig. 2 is a processing flowchart in the patient positioning system according to the first embodiment of the present invention;
Figs. 3A and 3B are explanatory views (radiographs) showing respectively a state where reference points are input on an image displayed at a scale-down factor of 25% and a state where computing regions are set on an image displayed in size of 100%;
Fig. 4 is a representation showing a pixel array in the computing region on the image displayed in size of 100%;
Fig. 5 is a representation showing a pixel array in the computing region for explaining a representative-point computing method according to the present invention;
Fig. 6 is an illustration for explaining a weighting manner for each computing point in the computing region shown in Fig. 5;
Fig. 7 is a processing flowchart in a patient positioning system according to a second embodiment of the present invention; and
Figs. 8A and 8B are explanatory views (radiographs) showing respectively a state where computing regions are inputted on an image displayed in size of 100% and a state where representative points are set on the image displayed in size of 100%.

### DESCRIPTION OF THE PREFERRED EMBODIMENTS

Preferred embodiments of the present invention will be described below with reference to the drawings.

### (First Embodiment)

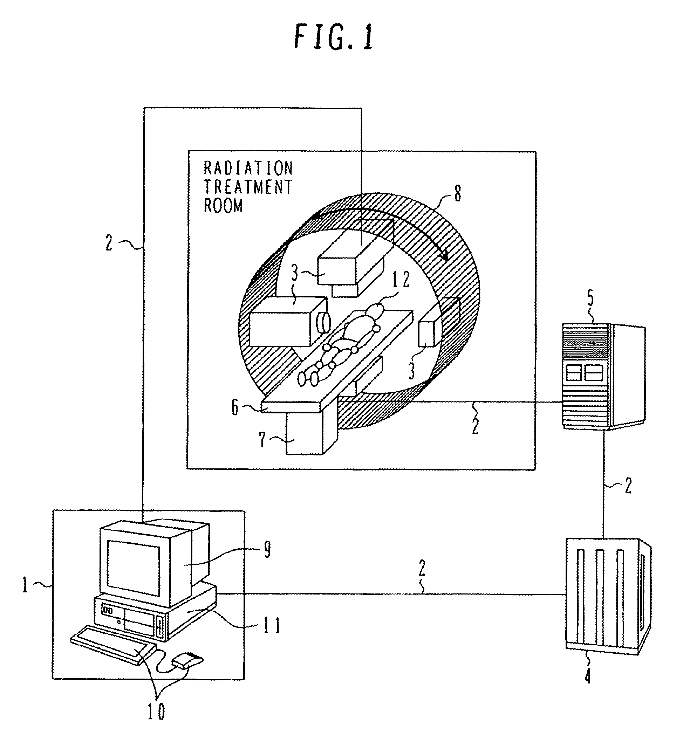

Fig. 1 schematically shows the overall configuration of a patient positioning system according to a first embodiment of the present invention.

As shown in Fig. 1, the patient positioning system of this embodiment is assumed to be used in treatment equipment using a radiation (e.g., a proton beam) and including a rotating gantry. In addition to such treatment equipment, the present invention is also applicable to other general types of radiation treatment equipment, including X-ray treatment equipment.

Further, as shown in Fig. 1, a patient positioning computer 1 is connected to an X-ray image capturing device 3, a medical image server (image information storage) 4, and a treatment bed controller 5 via a network cable 2. The treatment bed controller 5 controls a bed driving unit 7 for driving a bed (treatment bed) 6 on which a patient 12 lies when radiation is irradiated to the patient for treatment, thereby controlling the position of the bed 6. The X-ray image capturing device 3 captures an image of an affected area in the body of the patient 12 prior to the treatment irradiation in a state where the patient 12 is lying on the bed 6. The X-ray image capturing device 3 is located inside a rotating gantry 8 near the patient 12 and is installed to be rotatable around the patient 12 with rotation of the rotating gantry 8. Image data (second image information) captured by the X-ray image capturing device 3 and including the affected area in the body of the patient 12 is outputted, as a comparative image (DR (Digital Radiograph) image), to the patient positioning computer 1 via the network cable 2. On the other hand, a CT image (three-dimensional image) and a reference image (DRR (Digital Reconstructed Radiograph) image), which is a tomographic image appropriately produced from the CT image, are stored in the medical image server 4 and are obtained by the patient positioning computer 1 as required. The patient positioning computer 1 includes a monitor (display) 9 for displaying the comparative image obtained from the X-ray image capturing device 3 and the reference image obtained from the medical image server 4, an input device 10, such as a mouse or a keyboard, through which a computer operator makes various input operations, and a computing unit (corresponding to an enlarged region setting processor, a representative region setting processor, and a processing unit (means)) 11.

While the above description is made as transmitting the comparative image directly from the X-ray image capturing device 3 to the patient positioning computer 1, the image data may be transmitted to and stored in the medical image server 4 from the X-ray image capturing device 3, and the stored image data may be read, as the comparative image, out of the medical image server 4 by the patient positioning computer 1.

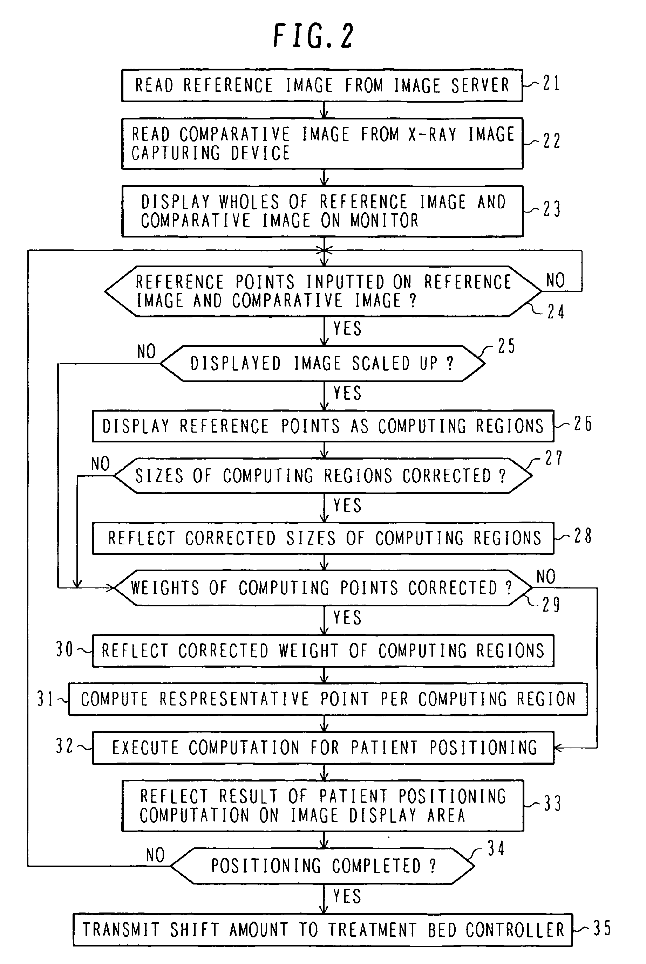

Fig. 2 shows a control flow executed by the computing unit 11 of the patient positioning computer 1. Steps of positioning the patient 12, executed by the patient positioning system of this embodiment, will be described below with reference to Fig. 2. The control flow is started when a positioning start instruction (e.g., an instruction input entered through the input device 10) is given from the operator.

First, the reference image prepared in the treatment planning stage and stored in the medical image server 4 is read into, e.g., a not-shown storage (memory) (step 21).

Then, the comparative image of the patient 12, which is captured by the X-ray image capturing device 3 in the state of the patient 12 lying on the bed 6, is read into, e.g., a not-shown storage (memory) from the X-ray image capturing device 3 (step 22). The comparative image is also transmitted from the X-ray image capturing device 3 (or the patient positioning computer 1) to the medical image server 4 and stored therein.

The above steps 21 and 22 may be replaced in order or may be executed in parallel.

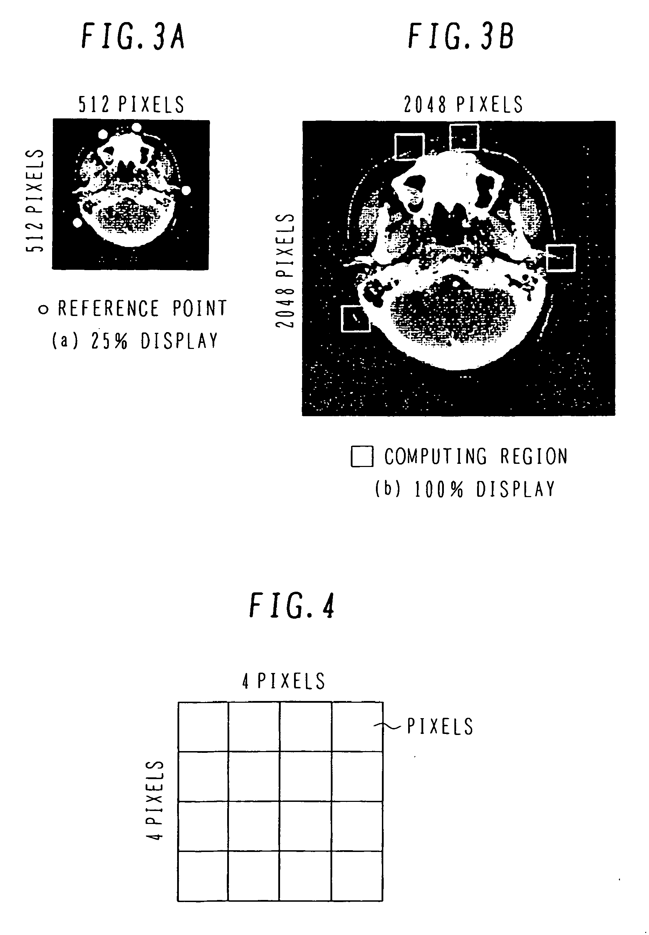

After the completion of read of both the reference image and the comparative image in the above steps 21 and 22, those images are displayed on the monitor 9 of the patient positioning computer 1 (step 23). At that time, the reference image and the comparative image are displayed in scaled-down sizes, as required, such that the whole of both the images is contained in an image display screen of the monitor 9. In this embodiment, it is assumed, for example, that the reference image and the comparative image are each an image having a size of 2048 × 2048 pixels (see Fig. 3B) and an image display area in the monitor 9 of the patient positioning computer 1 has a size of 512 × 512 pixels for each of the two images. Accordingly, the reference image and the comparative image are each displayed in size scaled-down to 25% (see Fig. 3A). Note that Figs. 3A and 3B show one of the reference image and the comparative image.

Then, it is determined whether reference points (particular regions) have been inputted by the operator (step 24). More specifically, it is determined whether the operator has inputted reference points, which are used for the patient positioning, at respective corresponding positions in the reference image and the comparative image by using the input device 10 on the screen of the monitor 9 on which the whole of the reference image and the comparative image is displayed in size scaled down to 25%. The reference points are preferably set on the images in an entirely distributed way instead of being located in a portion. For that reason, the reference points are usually inputted by the operator in such a state that the whole of the affected area in the body of the patient is displayed. Clearly recognizable portions, e.g., tips of bones, are selected as positions to be inputted as the reference points.

Fig. 3A shows an example in which four reference points are inputted. As shown in Fig. 3A, the reference points are indicated by circle points.

Then, it is determined whether an image scale-up instruction has been inputted from the operator (step 25). More specifically, it is determined whether the operator has inputted an instruction for scaling up the image displayed on the monitor 9 by using the input device 10. If the scale-up instruction has been inputted, the reference point in the form of the circle point is scaled up and displayed as a computing region (enlarged region) in the form of, e.g., a rectangular box (step 26). This scaled-up state is shown in Fig. 3B. Fig. 3B shows the state where the image of 2048 × 2048 pixels is displayed in size of 100% (in fact, only 1/4 of the entire image is displayed on the monitor 9 in the 100% display state). As shown in Fig. 4, the computing region is constituted as a rectangular region of 16 pixels with one side containing 4 pixels. Stated another way, when scaling-up of the image displayed on the monitor 9 is instructed, the reference point formed by 1 pixel on the scaled-down image, shown in Fig. 3A, is enlarged depending on the scaling-up factor (or scaling-down factor) and is set as the computing region of 4 × 4 pixels, as shown in Fig. 3B.

Then, it is determined whether the set computing region has been corrected by the operator (step 27). In this embodiment, the operator is able to, e.g., expand/contract, move and rotate the computing region for correction thereof. If any correction has been inputted by the operator using the input device 10, the corrected computing region is reflected at once (step 28). While the computing region has a rectangular shape in this embodiment, the shape of the computing region is not necessarily limited to a rectangle and can be defined as any other suitable one including a circle or an ellipse.

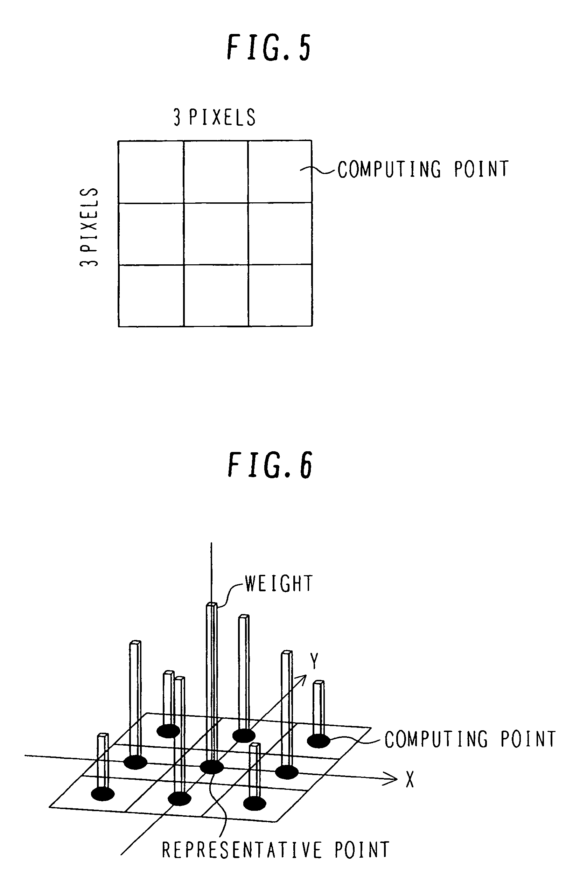

After the computing region has been decided in such a way, a representative point (representative region) is set in the decided computing region. A method of computing the representative point will be described below with reference to Figs. 5 and 6.

For the sake of simplifying the explanation, the computing region is assumed here to be a rectangular region made up of 9 pixels with one side containing 3 pixels, as shown in Fig. 5. This corresponds to the case where the operator inputs the reference point in the state of the reference image and the comparative image being displayed on the monitor 9 at a scaling-down factor of 33%, and then instructs scaling-up of each image to the full 100% size.

In this embodiment, it is assumed that the computing point is given by each of the pixels contained in the computing region, and the computing region is a set of 9 computing points. Weights are allocated to the computing points, respectively, and one representative point is computed based on the computing points contained in the computing region while taking into account the weights. The weights used for computing the representative point are provided by pixel values in the computing region. The reason is as follows. Generally, in the patient positioning process using an X-ray radiographic image, a definite structure, e.g., a bone, is set as the computing point for the patient positioning in many cases, and the definite structure, e.g., the bone, has a higher brightness (larger pixel value) on the X-ray radiographic image. Therefore, the magnitude of the pixel value can be regarded as an index representing the definite structure, e.g., the bone.

More specifically, the weight of the computing point where the pixel value is maximized in the computing region is set to 1, and the weights of the other computing points are set by normalizing their pixel values based on the maximum pixel value. Then, the representative point is computed by multiplying the weights of the computing points by the respective coordinate values of the same computing points and summing the multiplied results. The coordinate values of the computing points used at that time are defined with the center of the computing region set as the origin. By converting the pixel values in the computing region to weights and computing the representative point in consideration of a resultant weight distribution as described above, it is possible to reproduce the point which has been intended by the operator to input. Incidentally, such a weight distribution can be said as being a probability distribution that represents certainty of the input information (namely, the point having the maximum pixel value and given with the weight 1 is the point inputted by the operator with the highest probability).

Fig. 6 is an illustration showing one example of the weight distribution resulting from allocating weights to the computing points in the computing region shown in Fig. 5. The weight distribution shown in Fig. 6 corresponds to the case where a pixel at the center of the computing region has a maximum pixel value, four pixels adjacent to the center pixel in the vertical and horizontal directions have a second maximum pixel value, and the remaining pixels at four corners have a minimum pixel value. In this case, by multiplying the weights of the computing points by the respective coordinate values of the same computing points and summing the multiplied results, the center pixel is computed as the representative point due to symmetry of the respective pixel values.

Thus, with the computing method described above, the representative point is computed by automatically executing the weighting process. In this embodiment, the weights can be corrected by the operator as required. In other words, it is determined prior to computing the representative point whether a correction has been inputted by the operator on the weights of the computing points automatically set from the pixel value distribution in the computing region (step 29). More specifically, the weight distribution in the computing region is graphically displayed on the monitor 9 of the patient positioning computer 1, as shown in Fig. 6, such that the operator is able to modify the weights expressed in the form of bar graphs, for example, by using the input device 10 (such as the mouse). If a weight correction has been inputted by the operator while looking at the displayed graph, the corrected weights are reflected at once (step 30).

After the weights have been decided in such a way, the representative point is computed per the computing region by the above-described computing method (step 31). Then, computation for the patient positioning is executed (step 32). More specifically, an image shift amount for minimizing an error between the corresponding representative points in the reference image and the comparative image is computed by the least square method using the computed representative points.

After the end of the computation for the patient positioning, the comparative image is rotated and/or moved in accordance with the computed shift amount, and this result is reflected on the image displayed on the monitor 9 (step 33). It is then determined whether the end of the patient positioning has been instructed from the operator (step 34). If the operator has confirmed the result of the patient positioning on the image displayed on the monitor 9 and has instructed the end of the patient positioning by using the input device 10 based on the determination that there are no problems, a shift amount (positioning information) for the patient 12 (or the bed 6) is transmitted to the treatment bed controller 5 via the network cable 2 (step 35). Responsively, the treatment bed controller 5 executes control of the bed driving unit 7 such that the bed 6 is moved for precise positioning of the patient 12.

In the above description, the reference image and the comparative image correspond respectively to first image information and second image information that are stated in claims as image information containing an affected area in the body of the patient.

The above-described patient positioning system of this embodiment can provide advantages as follows.

When the known reference point input method is used to input the reference points in the state of the reference image and the comparative image being displayed on the monitor 9 in scaled-down size as in the embodiment described above, pixels contained in each enlarged reference point (i.e., an enlarged computing region) on the scaled-up image are all selected, some one of those pixels is set as a computing point, and a shift amount is computed by the least square method so that an error between the corresponding computing points in the reference image and the comparative image is minimized. With the known method, therefore, the pixel selected as the computing point is not definite, thus resulting in a risk that a different point on the image from the point intended by the operator is selected as the computing point and the positioning accuracy is deteriorated.

In contrast, with the patient positioning system of this embodiment described above, the reference point inputted by the operator is enlarged and displayed as the computing region depending on the display scaling-down factor of the monitor 9, and the representative point is computed by the computing method in consideration of the pixel value distribution (weight distribution) in the computing region. In other words, the representative point can be set taking into account the probability distribution that represents certainty of the input information entered by the operator in the relevant computing region. Therefore, the desired point can be reproduced which has been intended by the operator at the time of inputting the reference point on the scaled-down image. As a result, by computing a deviation amount of the patient position using the thus-set representative point, it is possible to eliminate uncertainty in the input information of the reference point, which has been caused due to the image scaling-down in the known reference point input method, and to increase the accuracy in the patient positioning.

Further, with this embodiment, as described above in connection with the step 27 in Fig. 2, the computing region having been automatically set depending on the scaling-up (or -down) factor of the image displayed on the monitor 9 can be optionally corrected to a more preferable region at the discretion of the operator. Also, in the step 30, the operator can optionally correct the weights of the computing points, which have been automatically set from the pixel values in the computing region. Accordingly, when the operator is a doctor, for example, the computing region and/or the weights can be corrected based on expert knowledge and judgment of the doctor, and the positioning accuracy can be further increased.

In addition, the representative point computing method of this embodiment requires just the operation process of multiplying the pixel value by the coordinate value for each of pixels in the computing region which is made up of only several to several tens pixels, and summing the multiplied results. The remaining operation is to compute, similarly to the known reference point input method, the shift amount by the least square method so that an error between the corresponding representative points in the reference image and the comparative image is minimized. Accordingly, the amount of computations required in this embodiment is greatly reduced in comparison with that required in the known image comparing method in which pixel values are compared for all of pixels in image areas of the reference image and the comparative image, each of the image areas having a predetermined size enough to recognize the feature specific to the images. As a result, it is possible to execute the computation for the patient positioning at a higher speed and to precisely position the patient in a shorter time than those required in the patient positioning process based on the known image comparing method.

Thus, this embodiment enables an improvement in the positioning accuracy and a cut in the positioning time to be realized at the same time.

### (Second Embodiment)

While the first embodiment represents the case where the operator inputs the reference point, this second embodiment represents the case where the operator inputs the computing region. More specifically, when the reference image and the comparative image are displayed on the monitor 9 at a scaling factor of 100% or larger (such as 150% or 200%), for example, there is a possibility that a pointed portion of the skeletal structure or the like, which can be definitely recognized in the image displayed in scaled-down size, is rounded due to the scaling-up and cannot be definitely recognized. This second embodiment is applied to that case. In other words, when the operator designates a region having a certain size and including a point intended by the operator, a representative point is computed from the designated region and the computation for the patient positioning is executed using the representative point.

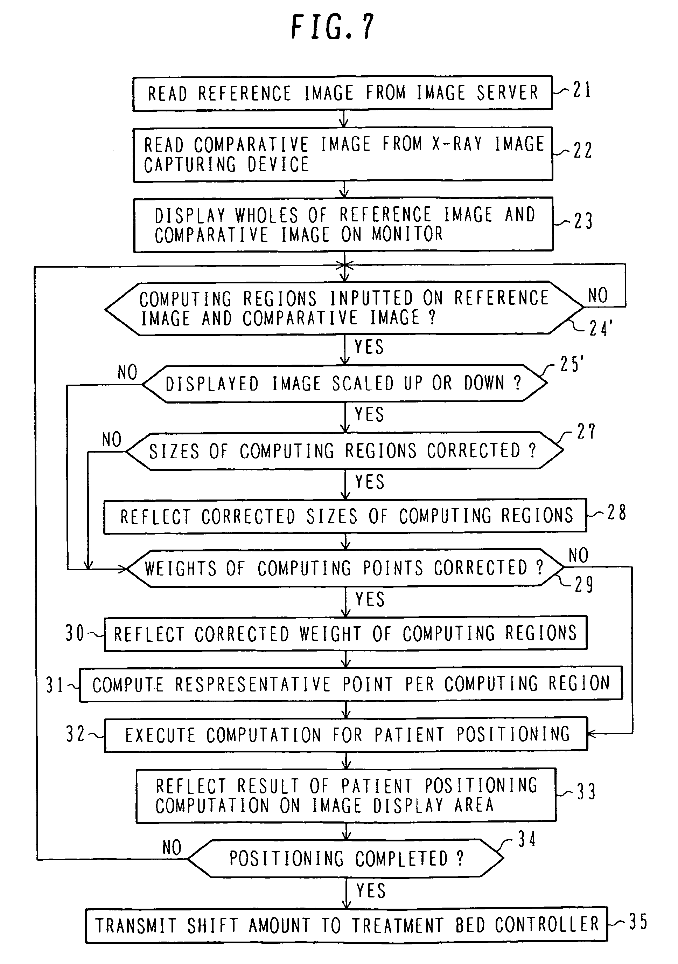

Fig. 7 shows a control flow executed by the computing unit 11 of the patient positioning computer 1 in this embodiment. Steps of positioning the patient 12, executed by the patient positioning system of this embodiment, will be described below with reference to Fig. 7.

Steps 21 - 23 are the same as those shown in Fig. 2. To describe briefly, the reference image and the comparative image are read from the medical image server 4 and the X-ray image capturing device 3, respectively, and are displayed on the monitor 9 of the patient positioning computer 1. In this state, the reference image and the comparative image are displayed in scaled-up sizes of 100% or larger, and only a part of one or both of the images is displayed on the monitor 9.

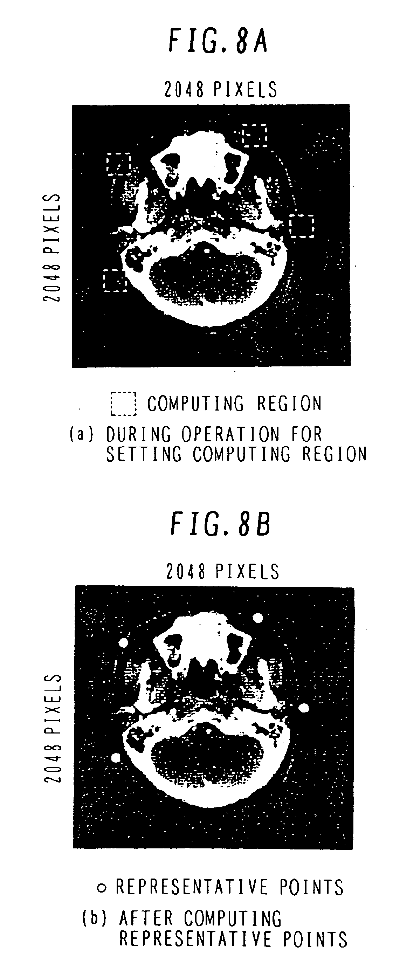

Then, it is determined whether computing regions have been inputted by the operator (step 24'). More specifically, it is determined whether the operator has inputted computing regions (each made up of plural pixels) in the form of rubber bands or the like, which are used for the patient positioning, at respective corresponding positions in the reference image and the comparative image so as to surround the intended portion by using the input device 10 (e.g., the mouse) on the screen of the monitor 9 on which a part of the reference image and/or the comparative image is displayed, while moving or changing over the images with manipulation of the input device 10. Fig. 8A shows an example in which four computing regions are inputted on the image displayed in size of 100%, and the computing regions are indicated by rectangular boxes. While each of the computing regions inputted by the operator has a rectangular shape in this embodiment, the shape of the computing region is not necessarily limited to a rectangle and can be defined as any other suitable one including a circle or an ellipse.

After the inputting of the computing regions, it is determined whether an image scale-up or -down instruction has been inputted from the operator (step 25'). More specifically, it is determined whether the operator has inputted an instruction for scaling down (or up) the image by using the input device 10 in order to confirm, e.g., the positional relationship of the inputted computing region on the entire image. If the scale-down (or -up) instruction has been inputted, it is then determined whether the computing region has been corrected by the operator (step 27).

Subsequent steps 28- 35 are the same as those shown in Fig. 2. More specifically, weights are allocated to respective computing points depending on corresponding pixel values in the computing region inputted by the operator (the weights are correctable), and a representative point is computed for each computing region by multiplying the weights by the coordinate values of the same computing points and summing the multiplied results. The image in this state is shown in Fig. 8B. Then, an image shift amount for minimizing an error between the corresponding representative points in the reference image and the comparative image is computed by the least square method. If there are no problems in the computed result, a shift amount of the patient 12 (or the bed 6) corresponding to the computed result is transmitted from the patient positioning computer 1 to the treatment bed controller 5. Responsively, the treatment bed controller 5 executes control to move the bed 6 for precise positioning of the patient 12.

The other processes in this second embodiment than described above are the same as those in the first embodiment, and hence a description thereof is omitted here.

With the second embodiment described above, by designating a region having a certain size and including a portion intended by the operator, the representative point is computed by the computing method in consideration of the pixel value distribution (weight distribution) in that region. Therefore, even when the reference image and/or the comparative image is displayed in scaled-up size on the monitor 9 and recognizability of the intended portion is deteriorated, it is possible to reproduce a point intended by the operator to be designated as the reference point. Accordingly, the accuracy in the patient positioning can be increased. Further, since the amount of computations is greatly reduced as in the first embodiment, a time required for positioning the patient can be cut. As a result, this embodiment also enables an improvement in the positioning accuracy and a cut in the positioning time to be realized at the same time.

While the second embodiment has been described in connection with the case of displaying the image on the monitor 9 in size of 100% or larger, the present invention is not limited to such an example. Namely, as a matter of course, the operator may designate the computing region in the state where the image is displayed in scaled-down size on the monitor 9. Such an application is very useful, for example, when the operator has poor eyesight and cannot properly input the reference points on the image displayed in scaled-down size on the monitor 9.

## Claims

1. A patient positioning system for positioning a patient undergoing radiation treatment, comprising:
a display (9) for displaying first image information and second image information containing an affected area in the patient's body;
an input device (10) for designating a particular region in the first image information and in the second image information displayed on said display (9); and
a processing unit (11) for producing positioning information used for positioning the patient based on position information of a representative region in the first image information and position information of the representative region in the second image information
**characterised by** a representative region setting processor for setting the representative region as a part of the designated particular region by computing respective weights for sub-regions in the particular region based on the pixel values distribution and summing the products of the weights of the sub-regions and coordinate values of the same sub-regions.

2. The system of claim 1, wherein said input device (10) is adapted to designate a computing region as said particular region, and said representative region setting processor sets said representative region as said computing region.

3. The system of claim 1, wherein
the display is capable of displaying at least one of the first and second image information in a scaled-down manner;
the input device is adapted to designate a reference point as said particular region;
an enlarged region setting processor is provided for setting an enlarged region obtained by scaling-up the reference point depending on the scaling-down factor; and
the representative region setting processor sets said representative region in the enlarged region.

4. The system of claim 3, wherein said representative region setting processor sets the representative region in the enlarged region in accordance with pixel information of the enlarged region, or distribution information of pixel values in the enlarged region.

5. The system of claim 2, wherein said representative region setting processor sets the representative region in the computing region in accordance with pixel information of the computing region, or distribution information of pixel values in the computing region.

6. The system of claim 1, wherein said representative region setting processor assigns a weight of 1 to one sub-region which has a maximum pixel value in the particular region and computes respective weights for other sub-regions in the particular region based on the weight 1 assigned to said one sub-region.

7. The system of claim 2, wherein said input device (10) is capable of individually inputting and setting the weights for the sub-regions in the enlarged region for each sub-region.

8. The system of claim 2, wherein said input device (10) is capable of individually inputting and setting the weights for the sub-regions in the computing region for each sub-region.

9. The system of claim 3, wherein said display (9) displays the enlarged region set by said representative region setting processor along with at least one of the first and second image information.

10. The system of claim 3 or 9, wherein said input device (10) is capable of correcting the enlarged region set by said representative region setting processor with input operation.

11. The system of claim 3, wherein said processing unit (11) produces the positioning information by the least square method such that a deviation between the position information of the representative region in the first image information and that in the second image information is minimized.

12. The system of claim 3, further comprising a treatment bed controller for controlling movement of a treatment bed in accordance with said position information.

13. The system of claim 3, further comprising an image information storage for storing at least one of the first and second image information.

14. A patient positioning method for positioning a patient undergoing radiation treatment by:
displaying first image information and second image information containing an affected area in the patient's body;
designating a particular region in the displayed first image information and second image information; and
producing positioning information used for positioning the patient based on position information of a representative region in the first image information and position information of the representative region in the second image information,
**characterised in that** the representative region is set as a part of the designated particular region by computing respective weights for sub-regions in the particular region based on the pixel values distribution and summing the products of the weights of the sub-regions and coordinate values of the same sub-regions.

15. The method of claim 14, wherein a computing region is designated as said particular region, and said representative region is set in said computing region.

16. The method of claim 14, wherein
said first and second image information are displayed in a scaled-down manner;
a reference point is designated in said particular region;
the reference point is enlarged depending on the scaling-down factor; and
the representative region is set in the enlarged region.

## Patentansprüche

1. System zum Positionieren eines Patienten bei einer Strahlungsbehandlung mit
einem Display (9) zur Darstellung erster Bildinformationen und zweiter Bildinformationen, die einen betroffenen Bereich im Körper des Patienten enthalten,
einem Eingabegerät (10) zum Bestimmen eines bestimmten Bereiches in den an dem Display (9) dargestellten ersten und zweiten Bildinformationen, und
einer Verarbeitungseinheit (11) zum Erzeugen von Positionierinformationen zum Positionieren des Patienten aufgrund von Positionsinformationen eines repräsentativen Bereichs in den ersten Bildinformationen und Positionsinformationen des repräsentativen Bereichs in den zweiten Bildinformationen,
**gekennzeichnet durch** einen Repräsentativbereichs-Einstellprozessor zum Einstellen des repräsentativen Bereichs als Teil des bestimmten speziellen Bereichs **durch** Berechnen jeweiliger Gewichte für Teilbereiche in dem speziellen Bereich aufgrund der Pixelwertverteilung und Aufsummieren der Produkte aus Gewichten der Teilbereiche und deren Koordinatenwerten.

2. System nach Anspruch 1, wobei das Eingabegerät (10) so ausgelegt ist, dass es als speziellen Bereich einen Rechenbereich bestimmt und der Repräsentativbereichs-Einstellprozessor den repräsentativen Bereich als den Rechenbereich einstellt.

3. System nach Anspruch 1, wobei das Display in der Lage ist, die ersten und/oder die zweiten Bildinformationen verkleinert darzustellen,
das Eingabegerät in der Lage ist, den speziellen Bereich als Bezugspunkt zu bestimmen,
ein Vergrößerungsbereichs-Einstellprozessor vorgesehen ist, um einen vergrößerten Bereich einzustellen, der durch Vergrößern des Bezugpunktes in Abhängigkeit von dem Verkleinerungsfaktor gewonnen wird, und
der Repräsentativbereichs-Einstellprozessor den repräsentativen Bereich in dem vergrößerten Bereich einstellt.

4. System nach Anspruch 3, wobei der Repräsentativbereichs-Einstellprozessor den repräsentativen Bereich in dem vergrößerten Bereich in Übereinstimmung mit den Pixelinformationen des vergrößerten Bereichs oder den Pixelwert-Verteilungsinformationen in dem vergrößerten Bereich einstellt.

5. System nach Anspruch 2, wobei der Repräsentativbereichs-Einstellprozessor den repräsentativen Bereich in dem Rechenbereich in Übereinstimmung mit Pixelinformationen des Rechenbereichs oder Pixelwert-Verteilungsinformationen in dem Rechenbereich einstellt.

6. System nach Anspruch 1, wobei der Repräsentativbereichs-Einstellprozessor einem Teilbereich, der einen maximalen Pixelwert in dem speziellen Bereich aufweist, das Gewicht 1 zuordnet und die jeweiligen Gewichte für die anderen Teilbereiche in dem speziellen Bereich aufgrund des dem besagten einen Teilbereich zugeordneten Gewichtes 1 berechnet.

7. System nach Anspruch 2, wobei das Eingabegerät (10) in der Lage ist, die Gewichte für die Teilbereiche in dem vergrößerten Bereich für jeden Teilbereich individuell einzugeben und einzustellen.

8. System nach Anspruch 2, wobei das Eingabegerät (10) in der Lage ist, die Gewichte für die Teilbereiche in dem Rechenbereich für jeden Teilbereich individuell einzugeben und einzustellen.

9. System nach Anspruch 3, wobei das Display (9) den von dem Repräsentativbereichs-Einstellprozessor eingestellten vergrößerten Bereich zusammen mit den ersten und/oder den zweiten Bildinformationen darstellt.

10. System nach Anspruch 3 oder 9, wobei das Eingabegerät (10) in der Lage ist, den von dem Repräsentativbereichs-Einstellprozessor eingestellten vergrößerten Bereich mit einer Eingabeoperation zu korrigieren.

11. System nach Anspruch 3, wobei die Verarbeitungseinheit (11) die Positionierinformationen nach der Methode der kleinsten Quadrate derart erzeugt, dass die Abweichung zwischen den Positionsinformationen des repräsentativen Bereichs in den ersten Bildinformationen und denen in den zweiten Bildinformationen minimal wird.

12. System nach Anspruch 3 mit einer Behandlungsbettsteuerung zum Steuern der Bewegung eines Behandlungsbettes entsprechend den Positionsinformationen.

13. System nach Anspruch 3 mit einem Bildinformationsspeicher zum Speichern der ersten und/oder der zweiten Bildinformationen.

14. Verfahren zum Positionieren eines Patienten bei einer Strahlungsbehandlung, wobei
erste Bildinformationen und zweite Bildinformationen dargestellt werden, die einen betroffenen Bereich im Körper des Patienten enthalten,
ein spezieller Bereich in den dargestellten ersten und zweiten Bildinformationen bestimmt wird,
Positionierinformationen zum Positionieren des Patienten aufgrund von Positionsinformationen eines repräsentativen Bereichs in den ersten Bildinformationen und Positionsinformationen des repräsentativen Bereichs in den zweiten Bildinformationen erzeugt werden,
**dadurch gekennzeichnet, dass** der repräsentative Bereich als Teil des bestimmten speziellen Bereichs durch Berechnen jeweiliger Gewichte für Teilbereiche in dem speziellen Bereich aufgrund der Pixelwertverteilung und Aufsummieren der Produkte aus den Gewichten der Teilbereiche und deren Koordinatenwerten eingestellt wird.

15. Verfahren nach Anspruch 14, wobei als spezieller Bereich ein Rechenbereich bestimmt und der repräsentative Bereich in diesem eingestellt wird.

16. Verfahren nach Anspruch 14, wobei
die ersten und die zweiten Bildinformationen verkleinert dargestellt werden, in dem speziellen Bereich ein Bezugspunkt bestimmt wird,
der Bezugspunkt in Abhängigkeit von dem Verkleinerungsfaktor vergrößert wird und
der repräsentative Bereich in dem vergrößerten Bereich eingestellt wird.

## Revendications

1. Système de positionnement de patient pour positionner un patient subissant une radiothérapie, comportant :
un afficheur (9) pour afficher des premières informations d'image et des secondes informations d'image contenant une zone atteinte dans le corps du patient,
un dispositif d'entrée (10) pour désigner une région particulière dans les premières informations d'image et dans les secondes informations d'image affichées sur ledit afficheur (9), et
une unité de traitement (11) pour produire des informations de positionnement utilisées pour positionner le patient sur la base d'informations de position d'une région représentative dans les premières informations d'image et d'informations de position sur la région représentative dans les secondes informations d'image,
**caractérisé par** un processeur d'établissement de région représentative pour établir la région représentative en tant que partie de la région particulière désignée en calculant des poids respectifs pour des sous-régions dans la région particulière sur la base de la distribution de valeurs de pixels et en additionnant les produits des poids des sous-régions et des valeurs de coordonnées des mêmes sous-régions.

2. Système selon la revendication 1, dans lequel ledit dispositif d'entrée (10) est adapté pour désigner une région de calcul en tant que ladite région particulière, et ledit processeur d'établissement de région représentative établit ladite région représentative en tant que ladite région de calcul.

3. Système selon la revendication 1, dans lequel
l'afficheur est capable d'afficher au moins l'une des premières et secondes informations d'image à une échelle réduite,
le dispositif d'entrée est adapté pour désigner un point de référence en tant que ladite région particulière,
un processeur d'établissement de région agrandie est prévu pour établir une région agrandie obtenue en augmentant l'échelle du point de référence en fonction du facteur de réduction d'échelle, et
le processeur d'établissement de région représentative établit ladite région représentative dans la région agrandie.

4. Système selon la revendication 3, dans lequel ledit processeur d'établissement de région représentative établit la région représentative dans la région agrandie conformément à des informations de pixels de la région agrandie, ou des informations de distribution de valeurs de pixels dans la région agrandie.

5. Système selon la revendication 2, dans lequel ledit processeur d'établissement de région représentative établit la région représentative dans la région de calcul conformément à des informations de pixels de la région de calcul, ou des informations de distribution de valeurs de pixels dans la région de calcul.

6. Système selon la revendication 1, dans lequel ledit processeur d'établissement de région représentative attribue un poids 1 à une sous-région qui a une valeur de pixel maximale dans la région particulière et calcule des poids respectifs pour d'autres sous-régions dans la région particulière sur la base du poids 1 attribué à ladite sous-région.

7. Système selon la revendication 2, dans lequel ledit dispositif d'entrée (10) peut entrer et établir individuellement les poids pour les sous-régions dans la région agrandie pour chaque sous-région.

8. Système selon la revendication 2, dans lequel ledit dispositif d'entrée (10) peut entrer et établir individuellement les poids pour les sous-régions dans la région de calcul pour chaque sous-région.

9. Système selon la revendication 3, dans lequel ledit afficheur (9) affiche la région agrandie établie par ledit processeur d'établissement de région représentative ainsi qu'au moins l'une des premières et secondes informations d'image.

10. Système selon la revendication 3 ou 9, dans lequel ledit dispositif d'entrée (10) peut corriger la région agrandie établie par ledit processeur d'établissement de région représentative avec une opération d'entrée.

11. Système selon la revendication 3, dans lequel ladite unité de traitement (11) produit les informations de positionnement par le procédé des moindres carrés de sorte qu'un écart entre les informations de position de la région représentative dans les premières informations d'image et celles dans les secondes informations d'image est minimisé.

12. Système selon la revendication 3, comportant de plus un contrôleur de lit thérapeutique pour commander le mouvement d'un lit thérapeutique conformément auxdites informations de position.

13. Système selon la revendication 3, comportant de plus une mémoire d'informations d'image pour mémoriser au moins l'une des premières et secondes informations d'image.

14. Procédé de positionnement de patient pour positionner un patient subissant une radiothérapie en :
affichant des premières informations d'image et des secondes informations d'image contenant une zone atteinte dans le corps du patient,
désignant une région particulière dans les premières informations d'image et secondes informations d'image affichées, et
produisant des informations de positionnement utilisées pour positionner le patient sur la base d'informations de position d'une région représentative dans les premières informations d'image et d'informations de position de la région représentative dans les secondes informations d'image,
**caractérisé en ce que** la région représentative est établie en tant que partie de la région particulière désignée en calculant des poids respectifs pour des sous-régions dans la région particulière sur la base de la distribution de valeurs de pixels et en additionnant les produits des poids des sous-régions et des valeurs de coordonnées des mêmes sous-régions.

15. Procédé selon la revendication 14, dans lequel une région de calcul est désignée en tant que ladite région particulière, et ladite région représentative est établie dans ladite région de calcul.

16. Procédé selon la revendication 14, dans lequel
lesdites premières et secondes informations d'image sont affichées à une échelle réduite,
un point de référence est désigné dans ladite région particulière,
le point de référence est agrandi en fonction du facteur de réduction d'échelle, et
la région représentative est établie dans la région agrandie.
